# EUROPEAN PATENT APPLICATION

(11) **EP 1 314 787 A2**
(43) Date of publication of application: **28.05.2003**
(21) Application number: 02026076.6
(22) Date of filing: 22.11.2002
(51) Int. Cl.: C12Q 1/68

(54) **The Midkine gene is associated with cancer**

(30) Priority: 26.11.2001 JP 2001359503
(71) Applicant: Kudoh, Norio, Tokyo 151-0061 (JP)
(72) Inventor: Shinozawa, Takao, Kumagaya-shi, Saitama (JP); Ahmed, Kazi Mokim, c/o Dpt. of Biological and, 1-5-1 Tenzincho, Kiryu-shi, Gunma (JP); Shitara, Yoshinori, Gunma-gun, Gunma (JP); Kuwano, Hiroyuki, Maebashi-shi, Gunma (JP); Takenoshita, Seiichi, Fukushima-shi, Fukushima (JP)
(74) Representative: Gerstein, Hans Joachim

(57) **Abstract**

A method of anticipating risk of the onset of cancer in an individual, including (1) obtaining a sample derived from the individual, (2) analyzing a polymorphism of a Midkine gene concerning the sample of (1), and (3) anticipating risk of the onset of cancer based on the polymorphism determined in (2) above.

## Description

The present invention relates to a gene associated with an onset of cancer, and a method of anticipating risk of the onset of cancer from a genotype.

Midkine (hereinafter, described as MK) is a heparin-binding protein which contains a large amount of basic amino acid and cysteine. A function thereof includes embryonal cell differentiation, the construction of cells, the growth of neural cell and the like. The function plays an important role in life activity. However, on the other hand, the expression of a MK gene in an adult is temporary and partial.

The association of the MK gene with cancer has been recently clarified. For example, it is described that the expression of the MK gene in cancer tissues such as Wilm's tumor, colorectal cancer, gastric cancer and lung and esophageal cancers becomes 10-fold of high expression rate in comparison with that in normal tissues (Cancer Res., 53, 1281-1285, 1993). Further, it is also reported that truncated MK mRNA(tMK mRNA) expresses in cancer tissues (Biochem. Biophys. Res. Commun., 219, 256-260, 1996, and Aridome, K., et al. Br. J. Cancer, 78, 472-477, 1998). A method of diagnosing cancer by the expression frequency analysis of a MK gene is also proposed (Jpn. Pat. Appln. KOKAI Publication No. 6-113898). However, the relation between a MK gene and mutation in this gene has not been cleared yet.

In consideration of the above-mentioned circumstances, an object of the present invention is to provide a method of anticipating risk of the onset of cancer by a mutation site in a MK gene which is associated with cancer and a genotype thereof.

The present invention is based on the finding by the present inventors that a polymorphic gene type which exists in the 62^{nd} nucleotide on intron 3 of a Midkine gene, namely the 808 position on the MK gene, is associated with an onset of cancer.

The present inventors have intensively studied based on the knowledge in order to attain the above-mentioned object, and have found the procedure below. That is, a method of determining risk of the onset of cancer in an individual, comprising:
(1) obtaining a sample derived from the individual;
(2) analyzing a polymorphism of a Midkine gene concerning the sample of (1); and
(3) determining risk of the onset of cancer based on. the polymorphism analyzed in (2) above.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a schematic diagram showing a gene map of a MK gene;
FIG. 2 is a flow chart showing one example of a anticipation method according to the present invention;
FIG. 3 is a block diagram showing one example of a anticipation device according to the present invention;
FIG. 4 is a flow chart showing another example of the anticipation method according to the present invention;
FIG. 5 shows an example of a table for use in the anticipation methods according to the present invention;
FIG. 6 is a view schematically showing the result of electrophoresis carried out for analyzing a polymorphic genotype associated with cancer; and
FIG. 7 is a view showing a secondary structure (namely, stem-loop structure) of an intron 3 region of MK gene pre mRNA.

### 1. Gene associated with cancer

The present inventors previously clarified the presence of a mutation and its genotype, and already reported it (Ahmed, K. M., Shitara, Y., Kuwano, H., Takenoshita, S., Ushimuro, K., and Shinozawa, T.,; Genetic variations of the midkine (MK) gene in human sporadic colorectal and gastric cancers, International journal of molecular medicine 6:281-287, 2000).

At this time, the present inventors compared the genotype of the mutation site in a cancer patient with the genotype of the mutation site in a normal person. Then, the association of the mutation gene with the onset of cancer have been clarified. In other words, the present inventors found for the first time that a mutation which exists at the 62^{nd} base on intron 3 of a Midkine gene is associated with the onset of cancer.

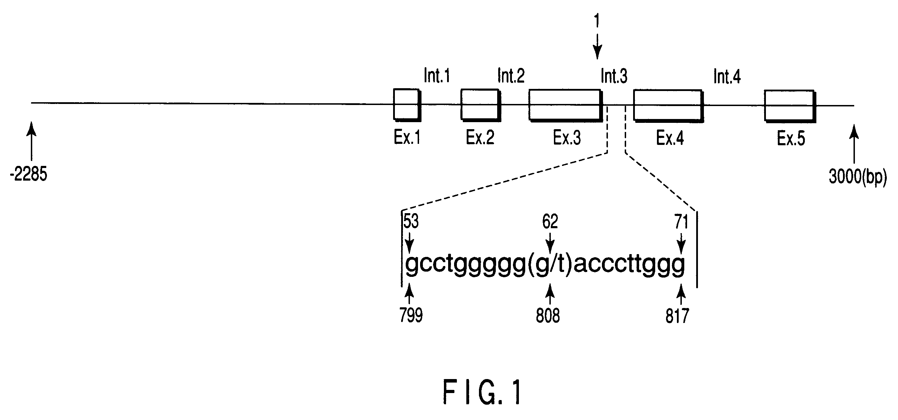

The MK gene is schematically shown in FIG. 1 (FIG. 1). A mutation associated with the onset of cancer exists in intron 3 (described as "Int.3" in FIG. 1). The mutation site corresponds to the 62^{nd} counted from the side of exon 3 (described as "Ex.1" in FIG. 1) of intron 3. Further, the site corresponds to the 808 position according to a usual declaration in which a starting site of transcription is +1. In the present specification, the mutation site associated with cancer according to the present invention is called as "MK808".

The genotype which can exist at the mutation site of MK808 is guanine (hereinafter, described as G or g) and thymine (hereinafter, described as T or t). In the wild type, both the genotypes of the alleles are G, namely G/G homozygote (hereinafter, described as G/G or G/G type). In the mutation type, there is a case that the genotype of either of alleles is T, namely G/T heterozygote (hereinafter, described as G/T or G/T type), and there is a case that both the genotypes of alleles is T, namely T/T heterozygote (hereinafter, described as T/T or T/T type). When a person whose this genotype of MK808 is G/T or T/T is susceptible to cancer in comparison with a person with G/G.

Further, when the genotype of MK808 is G/T heterozygote or T/T homozygote, it is considered that the stem loop structure of the pre mRNA from MK gene becomes unstable in comparison with a case of the wild type. As a result of unstableness of the secondary structure of this pre mRNA, it is considered that a short form MK mRNA(truncated MK mRNA, tMK mRNA) is generated. Accordingly, when it is anticipated that the tMK mRNA is generated, the person who has these genotypes is considered to be susceptible to cancer in comparison with a case of being not anticipated.

Specifically, the case in which it is anticipated that the short form (truncated) MK mRNA is generated is as following: that is, a case in which a genotype other than the wild type is observed in the allele in the any gene locus or a region (for example, intron 2, exon 3 and intron 3) of the MK gene including MK808. Further, a case in which a genotype other than the wild type is observed is, for example, a case in which any mutation is generated.

### 2. Term

The "gene associated with cancer" used herein is a gene which has an influence on the risk of onset of cancer. In other words, it is a gene whose genotype has an influence on risk of the onset of cancer. The term "genotype" used herein shows an existence state of the allele of the gene locus to be noticed.

The term of "MK808" used herein shows mutation which is the gene associated with cancer according to the present invention, namely a polymorphism. The terms of "polymorphism" and "polymorphic gene" used herein indicate a plurality of allele groups which occupy one gene locus, or individual alleles which belong to the allele groups. Further, since MK808 is single nucleotide substitution, its mutation is generally called as SNP (Single Nucleotide Polymorphism).

The term of "risk of the onset of cancer" used herein shows a degree of risk of the onset of cancer in an individual which is an objective. Accordingly, when the risk of the onset is high, the individual is susceptible to cancer.

The term of "cancer" used herein shows integrally various malignant tumor, namely cancer and sarcoma. Examples thereof include colorectal cancer, gastric cancer, esophageal cancer, laryngeal cancer, tongue cancer, lung cancer, hepatoma, pancreatic cancer, urocyst cancer, prostatic cancer, breast cancer and uterus cancer, etc. In particular, the association of mutation in MK808 or intron 2, exon 3 and intron 3 with the onset of colorectal cancer has stronger tendency than association with another cancer.

### 3. Method of anticipating risk of onset of cancer

According to the present invention, risk of the onset of cancer can be anticipated by utilizing the above-mentioned knowledge. The method of determining risk of the onset of cancer is within the range of the present invention.

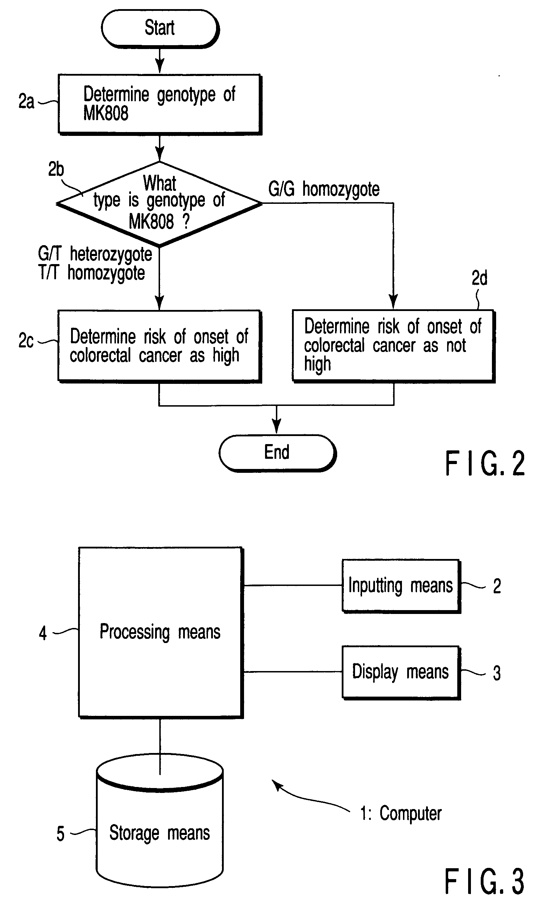

A case of colorectal cancer is illustrated as one example of the method of determining risk of the onset of cancer according to the present invention with reference to FIG. 2. All of operations below are manually carried out by hands of an operator.

The operator gathers a sample such as a blood sample or the like from an individual which is an objective of the anticipation and initiates the anticipation. In step 2a, processings such as purification and extraction are carried out to the collected sample as required, and thereafter, a genotype of MK808 in the sample is determined. Then, the process proceeds to step 2b.

In step 2b, it is determined whether the genotype of MK808 determined in step 2a is G/T or T/T or G/G. As a result of the determination in step 2b, when the genotype is G/T or T/T, the process proceeds to step 2c and when determined as G/G, the process proceeds to step 2d.

In step 2c, it is anticipated from the determination result of step 2b that the individual has the high risk of the onset of colorectal cancer. Then, all of the anticipation steps are terminated.

In step 2d, it is anticipated from the determination result of step 2b that the individual does not have the high risk of the onset of colorectal cancer. Then, all of the anticipation steps are terminated.

Means for determining the polymorphic genotype which can used in the present invention may be carried out using any means which is known itself. For example, nucleic acid containing an objective polynucleotide is prepared from a sample obtained from an objective individual, and the genotype may be determined.

The "individual" which is the objective of the method of the present invention may be an arbitrary mammal including a human, a dog, a cat, a cow, a goat, a pig, a sheep and a monkey, but a human, in particular, a Japanese is the most preferable individual.

The "sample" used herein means biological samples such as blood, serum, lymph, tissue, hair and earwax which are collected from a biological individual. Further, the "sample" may be a sample obtained by carrying out requisite arbitrary pretreatments such as homogenate and extraction to a biological sample, if necessary. The pretreatment can be selected by those skilled in the art in accordance with an objective biological sample.

The nucleic acid to be prepared from a sample may be prepared from DNA. For example, means for preparing a genome DNA sample from an objective can be carried out by any means which is usually used, such as a method of extracting from a hemocyte cell such as leukocyte, monocyte, lymphocyte and granulocyte in peripheral blood obtained from an objective, using a phenol-chloroform method, a salting-out method or a commercially available kit.

When the amount of polynucleotide is small, an operation of amplifying polynucleotide may be carried out as required. The amplification operation may be carried out, for example, by polymerase chain reaction (hereinafter, described as PCR in abbreviation) using enzymes such as DNA polymerase and the like.

According to requirement, the genotype of MK808 is determined after carrying out the extraction operation and/or the amplification operation.

Means for determining the genotype may carry out sequencing after cloning, and any means which is generally used such as a direct sequence method, a SSCP method, an oligonucleotide hybridization method and a specific primer method may be used. As an additional method of determining the genotype, there can be used other known methods such as a PCR-RFLP method for carrying out using restriction enzyme (PCR-restriction enzyme fragment length polymorphism method), a PCR-SSP (PCR-specific sequence primers) method, a PCR-SSO (PCR-sequence specific oligonucleotide) method which combines a dot plotting method and PCR, and a PCR-SSCP method. However, the method is not limited to these.

The genotype of MK808 is determined according to the above-mentioned methods, and the risk of the onset of colorectal cancer can be anticipated based on the result.

Further, the genotype of the MK808 is determined and a genotype of a gene associated with cancer which is known itself is determined, and thereafter the risk of the onset of cancer may be determined based on the results in a comprehensive manner. Moreover, the method is also within the range of the present invention. Even in such a case, based on the genotypes determined, for example, a table which shows information which corresponds the risk of the onset of cancer with the genotypes is investigated, the risk of the onset of corresponding cancer is extracted, and thereby the risk of the onset of cancer may be determined.

As described above, the onset of colorectal cancer is influenced by the genotype of the MK808. That is, it is possible to determine risk of the onset of colorectal cancer in an individual in high probability based on the genotype of the MK808. However, the onset of cancer is not only associated with the genotype of the MK808, but also the onset may be influenced by complex factors of other gene factors and various environmental factors. Accordingly, information of other gene factors may be added to be anticipated in addition to information related to the gene associated with cancer. For example, the information may be information related to other gene factors, and information related to environmental factors, for example, the ageing, the uptake tendency of foods, an article of luxury and the like. A table showing information which corresponds those factors with the risk of the onset of cancer may be used in case of such method. The determinion method is also included in the range of the present invention.

Further, the above-mentioned method according to the present invention can be changed within the range of the invention of the present application, in accordance with requirement.

Furthermore, the above-mentioned method according to the present invention can be also further analyzed using a computer.

In other words, the present invention provides a method of determining the risk of the onset of colorectal cancer in an individual using a computer, the method including:
(1) inputting by an operator a genotype of MK808 determined using a sample derived from the individual, into a computer;
(2) determining by the computer risk of the onset of cancer in accordance with a table which corresponds the risk of the onset of colorectal cancer with a genotype previously stored in storage means of the computer, based on the genotype inputted in (1) above; and
(3) showing by the computer the determination result obtained in (2) to the operator.

The method can be carried out, for example, as following.

FIG. 3 is a constitutional diagram showing one embodiment of a device for carrying out the method of the present invention. Processing means 4 is connected with inputting means 2, display means 3 and storage means 5. As shown in FIG. 3, a computer 1 being the present embodiment is at least equipped with the inputting means 2 for inputting data in the computer 1 by an operator, the display means 3 for displaying various information, the processing means 4 such as a processor and CPU for controlling the computer and carrying out various processings, and the storage means 5 for storing a program, a table and the like. The inputting means 2 may be, for example, a key board and a mouse.

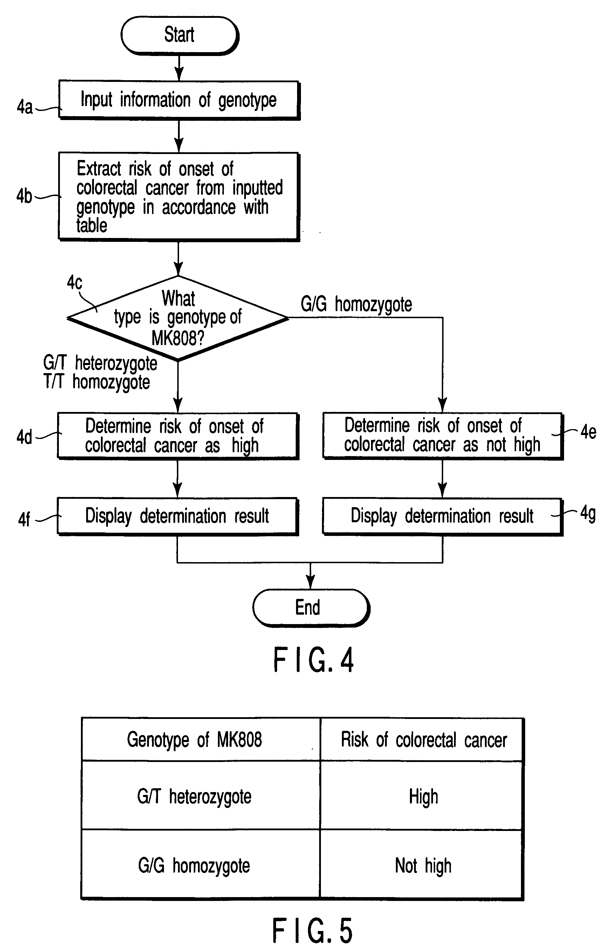

Example of the method of determining risk of the onset of cancer in an individual using a computer is illustrated below with reference to FIG. 4.

Hereat, the processing means 4 is a main control portion which collectively controls respective portions of the computer, carries out an anticipation program stored in a storage portion, and anticipates risk of the onset of cancer. When an operator inputs information of the genotype of the MK808 derived from an individual being an anticipation object, the inputted information is stored in the storage means 5 (in the FIG. 3), and transferred to step 4b. Hereat, the information of the genotype to be inputted may be a specific genotype and raw data, and it may be any information representing the information of the objective genotype.

In step 4b, the processing means 4 extracts the risk of the onset of corresponding colorectal cancer by reading out and investigating Table 1 (FIG. 5) which corresponds the risk of the onset of colorectal cancer with the genotype stored in the storage means 5 in advance, from the recorded information, and the process transfers to step 4c.

In step 4c, the processing means 4 determines the risk of the onset of colorectal cancer based on the result determined in step 4b. That is, when the genotype of the MK808 is G/T or T/T, it determines that the process transfers to step 4d, and when the genotype of the MK808 is G/G, it determines that the process transfers to step 4e.

In step 4d, the processing means 4 anticipates that the individual has a high possibility of being susceptible to colorectal cancer, and the process transfers to step 4f.

In step 4f, the processing means 4 displays in the display means 3 the result anticipated in step 4d, and/or records it in the storage means 5, and all of anticipation processes are terminated. The display hereat may be outputted as an image which indicates the risk of the onset of colorectal cancer previously stored in the storage means 5. Further, a loop which transfers from step 4f to step 4a may be set as required.

In step 4e, the processing means 4 anticipates that the individual has not a high possibility of being susceptible to colorectal cancer, and the process transfers to step 4g.

In step 4g, the processing means 4 displays in the display means 3 the result anticipated in step 4e, and/or records it in the storage means 5, and all of anticipation processes are terminated. The display hereat may be outputted as an image which indicates the risk of the onset of colorectal cancer previously stored in the storage means 5. Further, a loop which transfers from step 4g to step 4a may be set as required.

The "table" used herein is information which corresponds the risk of the onset of cancer with the genotype. For example, FIG. 5 shows an example in which Table 1 is shown as a chart. The table and chart used herein may be those which correspond the risk of the onset of cancer with the genotype. In other words, when it is a declaration which can practically show the correlation between the onset of cancer and the genotype, for example, "○", "×" and "Δ" may be well, or a score (for example, 1, 2, 3, 4 and 5 etc.) by a value simplified may be well.

Further, in the above-mentioned method, only MK808 is determined and inputted as information, and the anticipation is carried out. However, the anticipation may be carried out by considering another gene associated with cancer which is known itself, and/or environmental factors and the risk of the onset of cancer in addition to the information of MK808, and inputting as the information. In this case, the constitution of Table which is used in accordance with the information to be inputted may be changed.

Further, the above-mentioned method according to the present invention can be changed according to requirement so far as it does not deviate from the range of the invention of the present application.

Furthermore, a program for performing the method of anticipating risk of the onset of cancer in an individual using the above-mentioned computer is also within the range of the invention of the present application. The program is, for example, a program for, to anticipate the risk of the onset of cancer in an individual, making a computer function as:
(1) data recording means for recording information of a genotype of MK808 determined using a sample derived from the individual;
(2) means for analyzing risk of the onset of colorectal cancer in accordance with a table which corresponds the genotype with the risk of the onset of colorectal cancer previously stored in the computer, based on the genotype recorded in (1) above; and
(3) outputting means for outputting the anticipation result obtained in (2) above.

Further, the program may make the computer function as inputting means for inputting information of the genotype of the MK808 determined using a sample derived from the individual, in order to anticipate the risk of the onset of colorectal cancer in an individual. In addition, the program may be provided by being stored in any storage medium which is known itself.

Furthermore, an anticipating device for anticipating risk of the onset of colorectal cancer in an individual, comprising:
(1) storage means for storing a table which corresponds the genotype with the risk of the onset of colorectal cancer;
(2) inputting means for inputting information of a genotype of MK808 determined using a sample derived from an individual which is a tested object;
(3) anticipating means for anticipating the risk of the onset of colorectal cancer, based on the genotype inputted through the inputting means and the table stored by the storage means; and
(4) display means for displaying the anticipation result of the anticipation means is also within the range of the present invention.

Moreover, the present invention provides a method of anticipating risk of the onset of colorectal cancer in an individual using a computer, the method comprising:
(1) storing information of a genotype of MK808 determined using a sample derived from the individual, in data storing means;
(2) anticipating the onset of colorectal cancer in accordance with a table which corresponds the risk of the onset of colorectal cancer with a genotype, and which previously stored in the computer, based on the genotype stored in (1) above; and
(3) outputting the anticipating result obtained in (2) above.

As mentioned above, when it is anticipated that the short form MK mRNA is generated, it is considered that an object is susceptible to cancer in comparison with a case of being not anticipated. In the above-mentioned method, an example using MK808 is shown as the polymorphic site of the Midkine gene. However, as mentioned above, the risk of the onset of cancer may be similarly anticipated by detecting the mutation in another site and region of the MK gene. In this case, the polymorphic genotype which is determined by the above-mentioned methods, such as a method of anticipating risk of the onset of cancer and a method of anticipating risk of the onset of cancer in an individual using a computer, may be, for example, whether or not it is determined that the mRNA of MK becomes a short type. Further, the means for detecting the mutation may be carried out by any method which is known itself.

### 4. Polynucleotide for anticipating risk of onset of cancer

Further, the present invention provides a polynucleotide which can be used for anticipating risk of the onset of cancer.

The term "polynucleotide" used in the present specification means collectively a polynucleotide, an oligonucleotide and the like, for convenience. Further, "polynucleotide" hereat means a substance obtained by binding 2 or more nucleotides by phosphoric acid ester bonding. Nucleotide includes deoxyribonucleotide and ribonucleotide, but is not limited to these. Further, the polynucleotide, which is one embodiment of the present invention, may be a MK gene isolated from human, and DNA and RNA obtained based thereon, etc. Furthermore, the "polynucleotide" in the present invention denotes also artificially synthetic nucleic acids such as peptide nucleic acid, morpholino nucleic acid, methylphosphonate nucleic acid and S-oligonucleic acid.

The polynucleotide may be synthesized so as to have a desired sequence, and may be prepared from an organism sample. When the a polynucleotide is prepared from an organism sample, a sample is collected from an individual and then an operation of extracting the polynucleotide from the sample may be carried out. As a method of extracting a polynucleotide from an organism component, arbitrary extraction methods can be used in addition to a phenol extraction and an ethanol precipitation.

The polynucleotide provided, for the detection of MK808, according to the present invention can be used as a nucleic acid probe and a primer for determining a sequence of a polynucleotide of a sample derived from an individual.

For example, the nucleic acid probe may be used in a state of being provided at a substrate of a DNA micro array (in general, called as a DNA chip). A DNA micro array comprising the polynucleotide according to the present invention as the nucleic acid probe is also within the range of the present invention.

For example, the DNA micro array, which can be used in accordance with the present invention, is a device provided with a nucleic acid for detecting and/or analyzing the mutation on the MK gene, as a probe. And the DNA micro array for carrying out an analysis such as the detection of nucleic acid which is an object, is utilizing hybridization of the nucleic acid probe with nucleic acid to be detected. The DNA micro array can be produced by a method which is known itself.

The polynucleotide provided in accordance with the present invention is a polynucleotide which is comprised of or includes the following (a) to (o).
(a) A polynucleotide shown in SEQ ID No. 1 and SEQ ID No. 2.
(b) The polynucleotide shown in SEQ ID No. 3 and SEQ ID No. 4. These base sequences are not G or T by which the MK808 is usually detected, but sequences being A and C. These sequences can be preferably utilized as a probe for determining a sequence of a polynucleotide of a sample derived from an individual. For example, it is useful for enhancing the reliability of the result obtained when the genotype of the SNP site is analyzed.
(c) A modified polynucleotide in which one or more nucleotides excluding MK808 which is a gene associated with cancer are deleted, substituted or added, in the polynucleotides shown in (a) and (b) above.
(d) A polynucleotide which is a partial fragment of a MK gene containing a MK808 site. For example, the fragment may be about 10 nucleotides to about 500 nucleotides. It is preferably about 10 nucleotides to about 100 nucleotides. And when it is used as a nucleic acid probe, it is more preferably about 10 nucleotides to about 30 nucleotides.
(e) A complementary chain of the polynucleotide described in (a) to (d) above.

Further, so-called DNA micro array comprising the above-mentioned polynucleotide is also within the range of the present invention. The term "DNA micro array" used herein is used as a collective term containing not only a device which generally called as a DNA micro array, but also a device which generally called as a DNA chip.

The DNA micro array provided as one embodiment of the present invention is a device which can be used for the analysis of a gene in general. The basic structure of the DNA micro array according to one embodiment of the present invention is characterized in that the polynucleotide for detecting the mutation on the MK808 gene is provided on a substrate as a nucleic acid probe. Further, the DNA micro array can be produced by any one method which is known itself.

For example, the polynucleotide, which may be used as primer according to the present invention, may be a polynucleotide used for amplifying a polynucleotide for detecting or analyzing the mutation on the MK808 gene. The example of such primers may be polynucleotides which have a sequence shown in SEQ ID No. 5, SEQ ID No. 6, SEQ ID No. 7, SEQ ID No. 8, SEQ ID No. 9 and SEQ ID No. 10.

The present invention is further illustrated using Example.

### [Example]

### 1. Analysis of polymorphism of MK gene

### (1) Objective

As a group of cancer subjects, cancer subjects (98 cases of colorectal cancer, 60 cases of gastric cancer, 37 cases of breast cancer, 32 cases of lung cancer, and 51 cases of esophageal cancer) who are subject to treatment at the hospital in affiliation of Gunma University were used. As normal volunteers being a control group, 86 cases constituted by the staff members and students of Gunma University were used. All of the cancer subjects and normal volunteers are Japanese.

### (2) Extraction of genome DNA

Firstly, genome DNA's were extracted from samples collected from individuals of the respective groups.

In case of cancer subjects, tumor tissues cut from the cancer subjects by surgical operation carried out in the hospital in affiliation of Gunma University were used as samples. Further, the tumor tissues were portions which correspond to the central portion, the portion clearly determined as tumor, viewed from the whole tumor, and the portion clearly determined as being not tumor around tumor were used. The collected tissue samples were immediately frozen in liquid nitrogen after abscission, and preserved at -80°C.

After the tissues preserved in freeze were treated with sodium dodecylsulfate (hereinafter, described as SDS), Protenase K (manufactured by Wako Pure Chemicals, Co.), DNA's were extracted by carrying out phenol-chloroform extraction and ethanol precipitation.

Bloods were collected from normal volunteers as samples. The genome DNA was purified from peripheral blood collected, using a QIAamp Blood Kit (registered trademark, QIAamp Blood Kit, Qiagen, Hilden, Germany) according to a use manual.

### (3) PCR amplification of MK gene

The genome DNA extracted in (2) above was amplified by PCR using SEQ ID Nos. 5 to 8 as a primer. The amplification by PCR was carried out according to the following method. Fifty ng of the purified genome DNA obtained in (2) above was carried out in a final volume of 10 µL of a reaction solution which contains 0.2 µM each sense and antisense of a primer, 0.2 mM each of dNTP, 10 mM of Tris-HCl (pH = 8.8 at 25°C), 1.5 mM of MgCl₂, 0.5 U of Taq DNA polymerase (manufactured by Sawadi Co.), 10% dimethyl sulfoxide (DMSO, manufactured by Wako Pure Chemicals, Co.) (depending on a primer) and 0.1 µL of [α³²P]dCTP (about 3000 Ci/mmol, 10 mCi/ml, manufactured by Amasham Co.). The PCR reaction was carried out for 35 cycles composing following steps, at 95°C for one minute, 60 to 66°C for 40 seconds and 70°C for 50 seconds, in a DNA thermal cycler (PE, manufactured by Applied Biosystems Co.).

### (4) Polymorphism analysis of single stranded DNA by that high order structure

After the thermal modification of the PCR product amplified in (3), described above, was carried out at 94°C for 5 minutes in 85% formamide, it was cooled and subjected to electrophoresis. The electrophoresis was carried out for 3 to 4 hours under conditions of a temperature at 12°C and a constant output power of 25W or 40W using 5% poly(acryl amide) gel which contains 5% or 10% glycerol. After the electrophoresis, the gel was dried under vacuum, the auto radiography for the segregate DNA was carried out by exposing it on a Kodak XAR film at room temperature for a night.

### (5) Analysis of the nucleotide sequence of DNA

DNA fragments having a different degree of migration were extracted from the dried gel, and the PCR amplification for 35 cycles was carried out again under the same condition as that of (3) above using sense and antisense primer (respectively described in SEQ ID Nos. 9 and 10). The PCR product obtained is 357 base pair fragment (hereinafter, described as bp). Then, this fragment is incorporated into a vector using a TA cloning kit manufactured by Invitrogene Co., and the nucleotide sequence of DNA was determined using a DNA sequencer (DSQ 1000 type) manufactured by Shimadzu Corporation.

Further, for the simple detection of G/G and G/T polymorphisms, a method to test the breakage by restriction enzyme Ava II (New England Biolab, USA) followed by the electrophoresis in 1.5% agarose gel was carried out. The objective MK808 is in the restriction enzyme breakage sequence (5'-GGACC-3'). This breakage sequence is converted to non breakage sequence (5'-GTACC-3') by mutation from G to T. Accordingly, the polymorphic genotype could be determined as shown in the following result.

### 2. Result

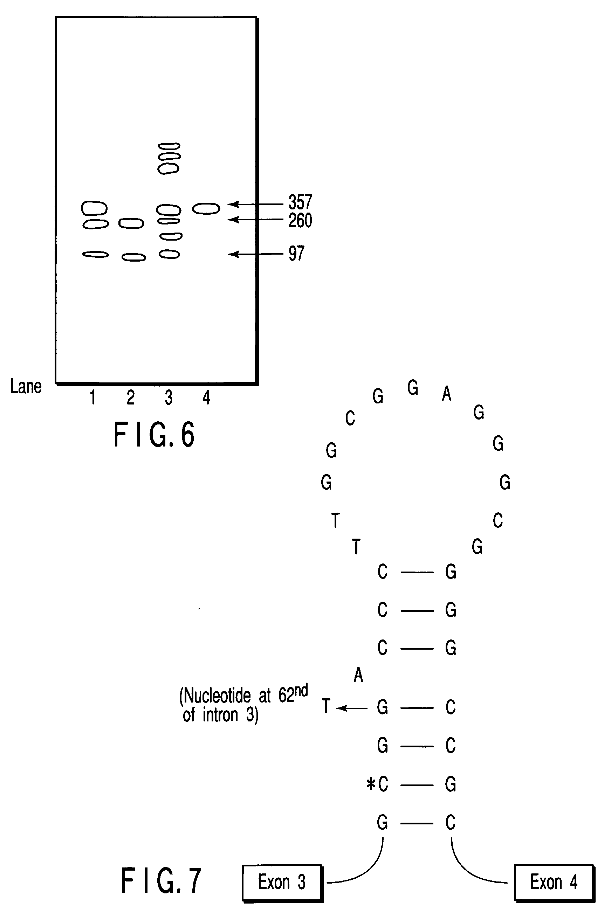

The electrophoresis result obtained in the result of (5) of item 1 above was typically shown in FIG. 6 (FIG. 6). FIG. 6 shows the results with respect to the lane 1 of G/T type colorectal cancer subjects, normal volunteers and the like, the lane 2 of G/G type colorectal cancer subjects, normal volunteers and the like, the lane 3 of DNA molecular weight standard (base pairs) and the lane 4 of G/G or G/T type colorectal cancer subjects, normal volunteers and the like, to which Ava II was not treated.

When the genotype of MK808 is G/G type, the recognition site of the restriction enzyme Ava II exists also in those derived from any of double stranded DNA of allele, for the PCR product obtained by PCR of (5) of item 1 above. Accordingly, when the genotype of MK808 is G/G type, 2 bands of 260bp and 97bp were detected. On the other hand, when the genotype of MK808 is G/T heterozygote, the recognition site of the restriction enzyme Ava II does not exist in the PCR product derived from one side of the double stranded DNA of allele. Accordingly, when the genotype of MK808 is G/T type, a band of 357bp was also detected in addition to 2 bands of 260bp and 97bp.

Table 1 shows the results obtained by the analysis as described above with respect to all of the cancer subjects and normal volunteers.

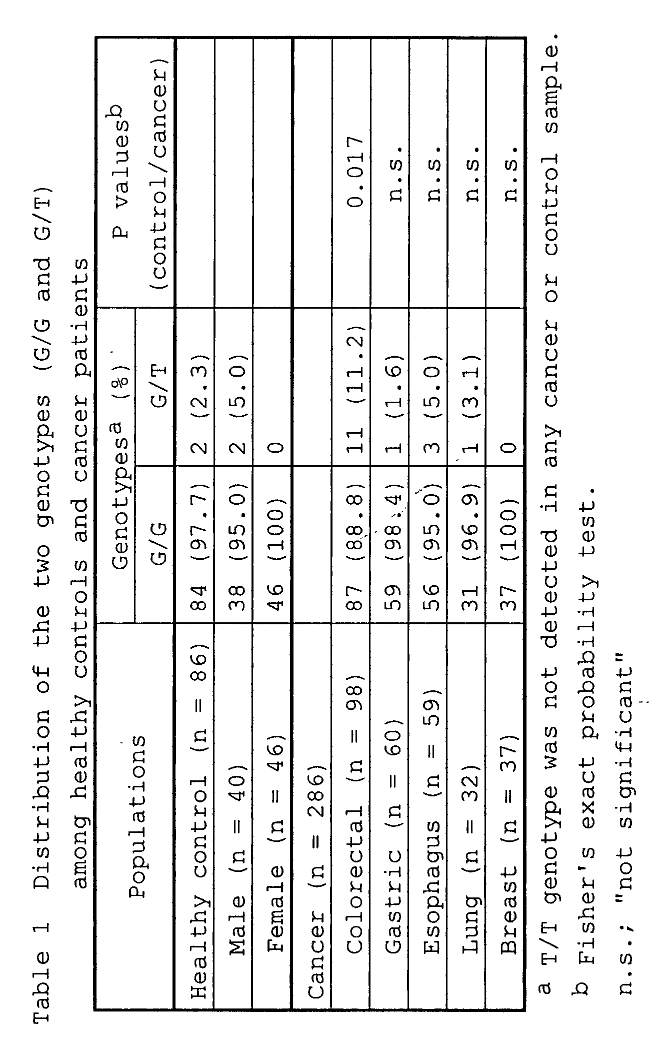

Table 1 shows the distribution and proportion of G/G and T/T of the respective genotypes of the normal volunteers and cancer subjects. The proportion of G/T type was 2.3% (2 cases in 86 cases) in normal volunteers. In the cancer subjects, colorectal cancer was 11.2% (11 cases in 98 cases), gastric cancer was 1.6% (1 case in 60 cases), esophageal cancer was 5.0% (3 cases in 59 cases), lung cancer was 3.1% (1 case in 32 cases), and breast cancer was 0% (0 case in 37 cases). From this result, it is obvious that G/T type in colorectal cancer was more frequent than the normal volunteer. Further, these results were analyzed statistologically by the Fisher's exact probability test method, and as a result, a significant difference between the group of colorectal cancer and that of control(P = 0.017) was detected. Further, colorectal cancer subjects were 11 cases (55%) among samples (20 cases) having G/T type, and to the contrary, the normal volunteers were 2 cases (10%). It was clarified that there was a significant relation between colorectal cancer and the genotype G/T of MK808. Further, in other cancers, although subjects having G/T type varies from 0 to 15%, the tendency of higher percentage in the cancer patiants than the normal volunteers was observed. Finally, a correlation of, mainly, colorectal cancer to the genotype G/T in MK808 was suggested.

### 3. Discussion

Recently, many studies have proved that growth factors not only promote the proliferation of a normal tissue but also induce malignant transformations (Aaronson, S. A., Science, 254: 1146-1153, 1991). Overexpression of growth factors has been found in many human tumors, and this phenomenon is often considered to be a cause of carcinogenesis (Aaronson, S. A., Science, 254: 1146-1153, 1991). The raise of expression of the MK gene is observed in human cancers (Aridome, K., Tsutsui, J., Takao, S., Kdomatsu, K., Ozawa, M., Aikou, T., and Muramatsu, T., Jpn, J. Cancer Res., 86:655-661, 1995). Further, a truncated form of MK mRNA with a deletion of exon 3 is found in cancer cells but not in normal cells (Kaname, T., Kadomatsu, K., Biochem. Biophys. Res. Commun., 219: 256-260, 1996). However, the biological function of MK during tumorigenesis has been not clarified.

The genetic polymorphism of the MK gene was identified by the present inventors by PCR-SSCP and DNA sequencing, and is already reported (Ahmed, K. M., Shitara, Y., Kuwano, H., Takenoshita, S., Uchinuro, K., and Shinozawa, T., Int. J. Mol., 6: 281-287, 2000). At this time, the present inventors clarified, for the first time, the association between the polymorphism of the MK gene and the risk of the onset of cancer. The present inventors also have carried out further assay for testifying the association between the genotype G/T of MK808 and colorectal cancer with respect to DNA's obtained from healthy persons of 86 cases, colorectal cancer of 98 cases, gastric cancer of 60 cases, esophageal cancer of 59 cases, lung cancer of 32 cases and breast cancer of 37 cases. The result was analyzed by statistical analysis using the Fisher's exact probability calculation method, and as a result, the association between G/T polymorphism and colorectal cancer was testified at a probability of P < 0.017.

Several studies of essential genes support the importance of the polymorphisms of intronic region. It is reported that single nucleotide polymorphisms in intron 12 of hHSH2 are associated with increase risk of colorectal cancer (Gessel, C., Plaschke, J., Pistorius, S., Hahn, M., Frank, S., Hampl, M., Gorgens, H., Koch, R., Saege, H. D., and Schackert, H. K. Eur. J. Cancer, 33: 1869-1874, 1997). Mavridou et al. reported that a polymorphism (G-to-A transition) located 61 nucleotides on intron 6 of the TP53 gene is associated with increased risk of ovarian cancer (Mavridou, D., Gornall, R., Campbell, I. G., and Eccles, D. M., Br. J. Cancer, 77: 676-677, 1998). Unlike mutations in exons, these intronic mutations do not usually result in aberrant expression of the genes. However, impact thereof on the cancer risk may be attributable to linkage with another susceptible gene (Sugimoto, K.M., Toyoshima, H., Sakai, R., Miyagawa, K., Hagiwara, K., Ishikawa, F., Takaku, F., Yazaki, Y., and Hirai, H., Blood, 79: 2378-2383, 1992).

As reported here, the present inventors have found that the heterozygous G/T polymorphism is significantly associated with an increased risk of colorectal cancer. This suggests that the polymorphism may contribute to the etiology of colorectal. This polymorphism was the only one, detected, genetic mutation in the exon 3 and intron 3 regions of the MK gene (Ahmed K. M., Shitara Y., Kuwano H., Takenochita S., Uchinuro K., and Shinozawa T., Int. J. Mol., 6:281-287, 2000). This polymorphism seems to be the cause of the aberrant splicing of MK mRNA.

In the wild-type MK pre-mRNA, a putative stem-loop structure is formed including the intron 3 polymorphism (FIG. 7). Free energy of this structure is ΔG = -21.9 Kcal/mol. It was calculated that the G-to-T transition destabilizes this stem-loop structure (that is, lowered from ΔG = -21.9 Kcal/mol to ΔG = -14.7 Kcal/mol) by disrupting a base-pairing interaction which is located at 62^{nd} (namely, MK808) on intron 3 in the stem 3. The destabilization of the secondary structure of the pre MK mRNA by the G/T polymorphism may confuse the splicing of this RNA leading to the production of tMK mRNA(and tMK protein). By this mechanism, it may be possible to explain the effect(or influence)of this polymorphism on the function of MK. Herein the above-mentioned free energy was determined by the genetic information processing software "Genetyx-win" (version 3.1).

## Claims

1. A method of anticipating risk of the onset of cancer in an individual, **characterized by** comprising:
(1) obtaining a sample derived from the individual;
(2) analyzing a polymorphism of a Midkine gene concerning the sample of (1); and
(3) anticipating risk of the onset of cancer based on the polymorphism determined in (2) above.

2. A method of anticipating risk of the onset of cancer in an individual according to claim 1, **characterized in that** analyzing a polymorphism of a Midkine gene described in (2) above is carried out by determining a genotype of the polymorphism.

3. A method of anticipating risk of the onset of cancer in an individual according to claim 1 or 2, **characterized in that** the polymorphism of the Midkine gene is a polymorphism existing in intron 2, exon 3 and intron 3.

4. A method of anticipating risk of the onset of cancer in an individual according to any one of claims 1 to 3, **characterized in that** when the polymorphism of the Midkine gene is different from a wild type, it is anticipated that the individual has high possibility of getting cancer.

5. A method of anticipating risk of the onset of cancer in an individual according to any one of claims 1 to 3, **characterized in that** when it is anticipated that mRNA of the Midkine gene is a truncated type, it is anticipated that the individual has high possibility of getting cancer.

6. A method of anticipating risk of the onset of cancer in an individual according to any one of claims 1 to 3, **characterized in that** when the polymorphism of the Midkine gene is MK808, and the genotype is G/T heterozygote or T/T homozygote, it is anticipated that the individual has high possibility of getting cancer.

7. A method of anticipating risk of the onset of cancer in an individual according to any one of claims 1 to 6, **characterized in that** the cancer is colorectal cancer.

8. A method of anticipating risk of the onset of cancer in an individual using a computer, the method comprising:
(1) recording information of a genotype of a Midkine gene determined using a sample derived from the individual in data recording means;
(2) directing risk of the onset of cancer based on the genotype recorded in (1) above, in accordance with a table which corresponds the risk of the onset of cancer with a genotype previously stored in the computer; and
(3) outputting the anticipation result obtained in (2) above.

9. A method of anticipating risk of the onset of cancer in an individual using a computer, the method comprising:
(1) inputting by an operator the genotype of the Midkine gene determined using the sample derived from the individual, into the computer;
(2) directing by the computer risk of the onset of cancer in accordance with a table which corresponds the risk of the onset of cancer with a genotype previously stored in storage means of the computer, based on the genotype inputted in (1) above; and
(3) showing by the computer the anticipation result obtained in (2) above to the operator.

10. A program for, to anticipate risk of the onset of cancer in an individual, making a computer function as:
(1) means for recording information of a genotype of a Midkine gene determined using a sample derived from the individual;
(2) means for directing risk of the onset of cancer based on the genotype recorded in (1) above, in accordance with a table which corresponds the risk of the onset of cancer with a genotype previously stored in the computer; and
(3) means for outputting the anticipation result obtained in (2) above.

11. An anticipating device anticipating risk of the onset of cancer in an individual, **characterized by** comprising:
(1) storage means for storing a table which corresponds a genotype with risk of the onset of cancer;
(2) inputting means for inputting information of a genotype of MK808 determined using a sample derived from an individual which is a tested object;
(3) anticipating means for inducing the risk of the onset of cancer, based on the genotype inputted through the inputting means and the table stored by the storage means; and
(4) display means for displaying the anticipation result of the anticipation means.

12. A DNA micro array comprising at least one polynucleotide which is selected from a group consisting of the following polynucleotides, as a nucleic acid probe to determine a genotype of MK808:
(a) a polynucleotide which is selected from SEQ ID No. 1, SEQ ID No. 2, SEQ ID No. 3 and SEQ ID No. 4;
(b) a modified polynucleotide in which one or more nucleotides excluding MK808 which is a gene associated with cancer are deleted, substituted or added, in the polynucleotides shown in (a) above;
(c) a polynucleotide which is a partial fragment of a MK gene containing MK808 site; and
(d) a complementary chain of the polynucleotides described in (a) to (d) above.


SEQUENCE LISTING
<110> GENE ASSOCIATED WITH CANCER
<120> Norio Kudoh
<130> 02S1288
<160> 10

<210> 1
<211> 19
<212> DNA
<213> Midkine gene

<400> 1
gcctgggggg acccttggg 19

<210> 2
<211> 19
<212> DNA
<213> Midkine gene

<400> 2
   gcctgggggt acccttggg 19

<210> 3
<211> 19
<212> DNA
<213> Midkine gene

<400> 3
   gcctgggggc acccttggg 19

<210> 4

<211> 19

<212> DNA

<213> Midkine gene

<400> 4

gcctggggga acccttggg 19

<210> 5

<211> 20

<212> DNA

<213> Midkine gene

<400> 5

gtcctgaact ctgttcctcg 20

<210> 6

<211> 20

<212> DNA

<213> Midkine gene

<400> 6

cacgcacccc agttctcaaa 20

<210> 7

<211> 21

<212> DNA

<213> Midkine gene

<400> 7

gcaactggaa gaaggagttt g 21

<210> 8

<211> 21

<212> DNA

<213> Midkine gene

<400> 8

acttggaatt gtcccctcac a 21

<210> 9

<211> 20

<212> DNA

<213> Midkine gene

<400> 9

gtcctgaact ctgttcctcg 20

<210> 10

<211> 20

<212> DNA

<213> Midkine gene

<400> 10

cacgcacccc agttctcaaa 20